# EUROPEAN PATENT SPECIFICATION

(11) **EP 1 366 468 B1**
(45) Date of publication and mention of the grant of the patent: **24.10.2007**
(21) Application number: 02700967.9
(22) Date of filing: 28.02.2002
(51) Int. Cl.: G06T 11/00, G01N 33/537, G01N 1/42

(54) **METHOD FOR LOCALIZING AND IDENTIFYING EPITOPES**
VERFAHREN ZUM LOKALISIEREN UND IDENTIFIZIEREN VON EPITOPEN
PROCEDE DE LOCALISATION ET D'IDENTIFICATION D'EPITOPES

(30) Priority: 05.03.2001 SE 0100728
(43) Date of publication of application: 03.12.2003
(73) Proprietor: Sidec Technologies AB, 171 77 Stockholm (SE)
(72) Inventor: SKOGLUND, Ulf, S-141 39 Huddinge (SE); VISA, Neus, S-141 39 Huddinge (SE)
(74) Representative: Westman, Per Börje Ingemar
(86) International application number: PCT/SE2002/000354
(87) International publication number: WO 2002/071336

(56) References cited:
- WO-A1-97/33255
- US-A- 5 241 471
- US-A- 5 340 749
- MIKEL VALLE ET AL.: 'Selection of antibody probes to correlate protein sequence domains with their structural distribution' PROTEIN SCIENCE vol. 8, 1999, pages 883 - 889, XP002950416
- ANNELIESE RAAB ET AL.: 'Antibody recognition imaging by force microscopy' NATURE BIOTECHNOLOGY vol. 17, September 1999, pages 902 - 905, XP002950417
- FRANCESC MIRALLES ET AL.: 'Electron tomography reveals posttranscriptional binding of Pre-mRNPs to specigic fibers in the nucleoplasm' THE JOURNAL OF CELL BIOLOGY vol. 148, no. 2, January 2000, pages 271 - 282, XP002950418
- MANFRED AUER: 'Three-dimensional electron cryo-microscopy as a powerful structural tool in molecular medicine' J. MOL. MED. vol. 78, 2000, pages 191 - 202, XP002950419
- PATENT ABSTRACTS OF JAPAN & JP 02 118 431 A (NIPPON TELEGR & TELEPH CORP) 02 May 1990
- PATENT ABSTRACTS OF JAPAN & JP 2001 004631 A (SHOKUHIN SYNGYO CENTER) 12 January 2001

## Description

### TECHNICAL FIELD

This invention relates to a method for three-dimensional structural analyses of macromolecules and multi-molecular complexes *in vitro* and *in situ* and for analyzing the structure in its physiological context.

### BACKGROUND

In order to understand the function of molecules from a mechanistic point of view, it is of great importance to gain knowledge about the structure. During the last 20 years, a number of structures of proteins and complexes of proteins and nucleic acids have been determined, mainly by NMR and X-ray crystallography. In general, when studying typical protein molecules, the data provided by these methods usually gives a resolution better than 3Å. When it comes to large-scale molecules and assemblies thereof, the resolution becomes much worse and in many cases it is not possible to study these large-scale structures. For structural studies using these methods, it is also necessary to bring the object under study out of its biological context (e.g. a cell).

By using transmission electron microscopy, it is possible to visualize, in two dimensions, supramolecular assemblies such as ribosomes or spliceosomes. A variant of electron microscopy, electron microscope tomography (Auer, J. Mol. Med., 78: -191-202, (2000)), can be used to generate a three dimensional image of an object. A detailed three dimensional reconstruction of the object has been practically impossible to accomplish due to the high electron radiation (typically ca 2000 e⁻/Å²/dataset) required.
Due to the low resolution achieved when making a 3D reconstruction, it is difficult to associate parts of the structure with a specific function.

A transmission electron microscopy method is available for 2D and/or 3D reconstructions of single objects by averaging after classification. This method can give a resolution of about 1 nm. This type of approach has been applied by Valle et al. (1999) to map specific domains of the connector protein of bacteriophage φ29 in 2D at about 3 nm resolution. This approach has some limitations. First, the molecules analyzed are directly attached to a surface which might cause alterations in their structural properties. Second, the single particle reconstruction method cannot resolve objects smaller than 100 kDa (Henderson, 1995). Third, this method provides average structures and consequently is not useful for analyses of structures in situ, or analyses of individual supramolecular complexes.
An alternative method for imaging antibody recognition has been proposed by Raab et al. (1999). In this case, the specimen under study is also attached to a surface. According to Raab et al., it should be possible to accomplish epitope mapping at the nanometer scale by using dynamic force microscopy. However, the details of how such a result can be achieved are not presented.

Recently, an algorithm for dramatically increasing the signal-to-noise ratio in EM-experiments was described (SE C 511 737). The algorithm is applied in a computer program, COMET. This allows studies using low dose electron radiation and the sample is thereby much less affected by the radiation and therefore in a state that is less perturbed by the experiment. Still, the resolution obtained with this method is too low to allow for unambiguous identification of specific parts of the object.

### SUMMARY OF THE INVENTION

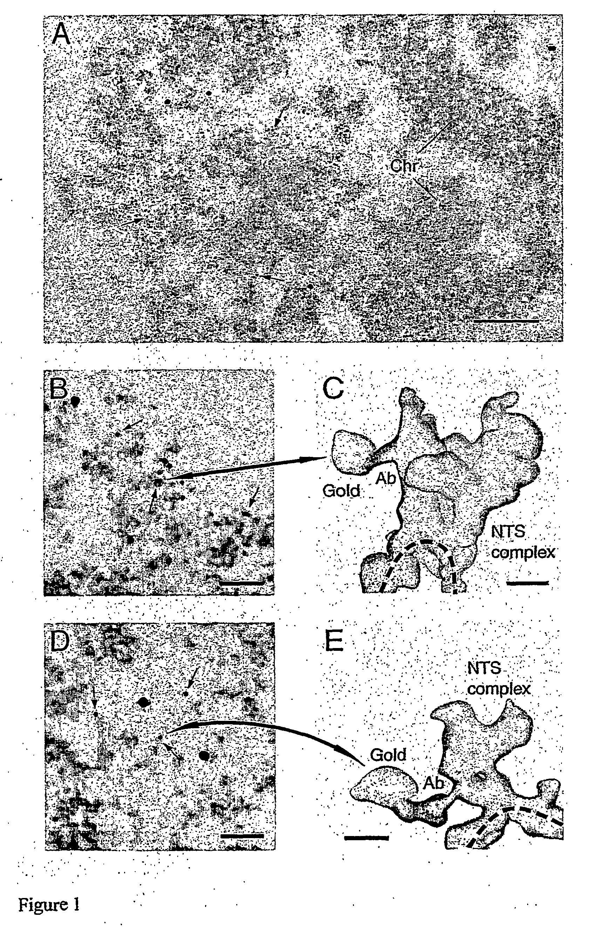

An object of the invention is to provide a method to make a detailed three-dimensional reconstruction of a macromolecular object and to determine where specific epitopes are located on this object. The invention as described herein can be used for identification of epitopes in their physiological context by labeling the epitopes *in situ,* or the epitopes can be identified *in vitro.* Identification of the epitopes involves the use of affinity reagents, typically antibodies, specific for a part of the molecule of interest, which may or may not be conjugated with small electron dense markers, together with electron tomography and the COMET algorithm. The use of COMET allows for low electron doses when taking EMT images. Somewhat surprisingly, these low doses do not disturb the interaction between the affinity reagent and its epitope. The method according to the invention allows a precise localization and identification of the epitope in the 3D reconstruction of an individual molecule or supramolecular complex, thus satisfying a long felt need. The ability of the method to pinpoint the location of specific epitopes is exemplified in figure 1C.

### DETAILED DESCRIPTION OF THE INVENTION

The present invention relates to a method for structural studies of macromolecular structures such as protein, nucleic acids or complexes or assemblies of one or more of these.

Four crucial aspects can be discerned:
1) Use of low dose electron radiation.
2) Labeling the object under study with affinity reagents.
3) Analysis of individual objects rather than averages of a multitude of images
4) Analysis of objects in situ.

The preparation of the object under study is based on either of the following procedures:
1) In the *in vitro* case, the object to be analyzed is a molecule or multimolecular complex in solution (e.g. in buffer). The affinity reagent (AR) is added to the solution and allowed to bind to the target molecule. The sample (in this case a solution containing the object under study plus the AR) is then applied onto a grid and frozen under conditions where no ice-crystals are formed (e.g. vitrification by plunging into liquid ethane). The frozen sample is then ready for data collection.
2) In the *in situ* case, the object under study is a molecule or multimolecular complex in a functionally relevant context (e.g. in a cell). The object under study has to be labeled with AR and sliced to obtain 25nm-1µm (preferably 70-90nm). Labeling and slicing can be envisioned to be performed in different order. In one case, the labeling is performed prior to the slicing. This enables the investigator to label objects in a living cell or unperturbed specimen prior to the slicing. In another case, the slicing is performed prior to the labeling. In this case, the slices are labeled with the AR.

In the case where labeling is performed prior to slicing (e.g. by injection of affinity reagents into a cell), the next step may be to fix and embed the specimen in a hydrophobic or, preferably, hydrophilic substance, or, alternatively, the specimen may be fixed and frozen.
The embedded or frozen cell is then sliced in a procedure termed ultra-microtomy, yielding so-called thin sections, with a thickness between typically 20 and 100 nm. In the case of frozen cells, the slicing procedure is termed cryo-microtomy, and the resulting section cryo-section. The cryo-microtomy is performed under conditions such as that the water in the sample is kept in amorphous form, not being allowed to form ice crystals. This can be achieved by freezing the specimen in the presence of a cryo protectant, typically sucrose.

The sample is then subjected to an electron beam. During data collection, several images are recorded of the sample, each image from a slightly different angle. These images form a so-called tilt-series. In the in vitro case, data collection should be performed at low temperature to-maintain the amorphous state of the sample. In the in situ case, data collection may be performed either at low temperature or at room temperature. The data collected in such a fashion is then processed, using standard software (Skoglund et al., 1996b). These procedures are well known to a person skilled in the art.

The different embodiments of the invention are characterized in claims 1-13.

It should be noted that the procedure in the above mentioned embodiments allows for studying samples of molecules in solution or in their biological in vivo environment.

In one embodiment of the invention, affinity reagents are bound to the object under study. These affinity reagents are visualized using electron microscopy and the epitope against which the antibody is directed can be located.
The advantage of using low dose radiation is that even non-covalently bound affinity reagents can be visualized together with its epitope. The affinity reagents may be either directed directly against the epitope of interest, so called direct labeling, or they may be directed against a primary affinity reagent already bound to the epitope of interest, this be termed indirect labeling.

The nature of the affinity reagent can vary widely, ranging from small molecules such as drugs or drug-like molecules and carbohydrates, via peptides, nucleotides, polypeptides, and polynucleotides to proteins, hormones and antibodies, the only condition being that the affinity reagent will not be displaced from its epitope by the electron dose prior to or during data collection. However, in the case of the affinity reagent being a small molecule, it may be needed to label it with e.g. an electron-dense marker or some other molecule that can be visualized in an electron microscope.

In another embodiment of the invention, an antibody labeled with an electron-dense marker is used as an affinity regent. The electron dense-marker can be readily visualized by the electron microscope.

In another embodiment, the size of the antibody may allow the detection of the antibody even in the absence of an electron-dense marker.

In yet another embodiment of the invention, a small molecule, such as a drug candidate (typically with a size of 200-600 Da) is labeled to an electron-dense marker. This may be used for localizing where on a macromolecule the small molecule may bind.

In another embodiment of the invention, a nucleotide, polynucleotide, peptide, polypeptide or protein may be used as an affinity reagent to enable the investigator to localize and identify the site of interaction between the probe (affinity reagent) and the object under study.

In another embodiment of the invention, two or more probes directed against different epitopes on the object under study. This may allow the investigator to study the relative positions of separate epitopes in one experiment.

In one embodiment of the invention, it may be favorable to increase the contrast in the images. This may be done by staining the sample with heavy atoms prior to data collection.

### EXAMPLE ON THE USE OF THE INVENTION

The following example is given for illustrating the results achieved by using the invention. The example presents the methods used and contains references so that a person skilled in the art can repeat the experiment. This will exemplify the invention used in the case of studying tissue *in situ.*

### Introduction

Splicing of pre-messenger RNA (pre-mRNA) is a complex process carried out by so called spliceosomes. Spliceosomes assembled *in vitro* have been investigated by electron microscopy in yeast (Clark et al., 1988) and in mammals (Reed et al., 1988). The spliceosome is assembled in an ordered and stepwise manner and is composed of a multitude of proteins and small nuclear RNAs (snRNAs).
The assembly process and the structure of spliceosomes *in vivo* are less well known. Splicing seems mainly to take place at, or close to, the active genes. Splicing components have been located to active genes, both by light microscopy (e.g., Amero et al., 1992; Wu et al., 1993; Zhang et al., 1994; Baurén et al., 1996; Neugebauer and Roth, 1997a) and by electron microscopy (Kiseleva et al., 1994; Puvion and Puvion-Dutilleul, 1996; Cmarko et al., 1999).

Several reports have established that the RNA polymerase II is involved in capping, splicing and polyadenylation (reviewed in Shuman, 1997; Corden and Patturajan, 1997; Steinmetz, 1997, Neugebauer and Roth, 1997b; Bentley, 1999). Combined, available data show that transcription and pre-mRNA processing are closely coupled in the cell nucleus and that the elongating RNA polymerase II should be present in a molecular complex that also contains the nascent transcript and different RNA processing factors.

In the model of co-transcriptional RNA processing, processing components, including splicing factors, bind to the RNA polymerase II (see Neugebauer and Roth, 1997b; Bentley, 1999). Several aspects of this model are however unclear and remain to be analyzed *in vivo.* It is unclear in the model where the RNA polymerase II picks up splicing factors and to what extent the elongating RNA polymerase carries splicing factors, particularly during transcription of multi-intron genes. To learn more about the structural basis of the coordinated transcription and splicing and answer the above questions, it is of great importance to study the *in vivo* structure of active genes.
Here, the active Balbiani ring 3 (BR3) gene in salivary gland cells of *Chironomus tentans* has been visualized and structurally characterized The BR3 gene is 10.9 kb long and encodes a secretory protein. 38 introns are approximately evenly spaced throughout the whole gene (Paulsson et al., 1990), and it is known that more than half of these introns are co-transcriptionally excised (Wetterberg et al., 1996). In this system, it is possible to identify the actively transcribing BR3 gene, in a complex termed granules, in the morphologically intact cell nucleus. This has enabled the use of electron tomography for reconstructing the three-dimensional (3D) structure of the active BR3 gene. It was demonstrated that splicing occurs within a defined, but dynamic, supramolecular complex.

Additionally and more importantly in the context of the invention, it is possible to localize where on the spliceosome the RNA polymerase and the U2 snRNP are located.
These results clearly demonstrate that the invention enables the investigators to pinpoint, by electron-tomography, where on a molecule a specific epitope is located.

Structural and immunological data show that each granule is a growing pre-mRNA transcript with its associated transcription and splicing components. Each such granule will be referred to as a " nascent transcript and splicing complex", NTS complex.

### The invention will be described with reference to the enclosed figures, in which:

Figure 1 demonstrates the immunolocalization of the U2 snRNP B" and the RNA polymerase II CTD in NTS complexes.
A), part of a section through the active BR3 gene locus. The 6 nm gold particles (arrows) show labeling with the anti-U2 snRNP B" antibody. Only actively transcribing BR3 gene segments are labeled. None or very few gold particles are seen over the inactive, compact, chromatin (Chr) in the right half of the picture. Four large gold particles are also seen. These are markers deposited on the preparation and used for alignment of the pictures in a tilt series, used for 3D reconstruction. Bar, 100 nm;
(B), projection of a 3D reconstruction of an area containing active BR3 gene segments, labeled with the anti-U2 snRNP B" antibody. The reconstruction has been made from low dose data with about 0.5 electrons/Å²/image. Arrows point out some of the 6 nm gold particles attached to the secondary antibody. One large gold particle, used for 3D reconstruction is also seen (top left). Bar, 50 nm;
(C), 3D reconstruction of the gold particle indicated in B (large arrow) and the labeled NTS complex, shown as surface rendering at 5 nm resolution. The antibody connection between the gold particle (yellow) and the NTS complex (blue) is shown in red. The dashed line indicates the chromatin axis. The 6 nm gold particle appears somewhat larger after reconstruction because it is a non-transparent object. Bar, 10 nm;
(D), projection of a 3D reconstruction of an area containing active BR3 gene segments, labeled with anti-RNA polymerase II CTD antibodies. The reconstruction has been made from low dose data with about 0.5 electrons/Å²/image. The arrows point out the 6 nm gold particles attached to the secondary antibody. Two large gold particles, used for 3D reconstruction, are also seen. Bar, 50 nm;
(E), 3D reconstruction of the gold particle indicated in D (large arrow) and the labeled NTS complex, shown as surface rendering at 5 nm resolution. The color-coding is as in C. The dashed line indicates the chromatin axis. Bar, 10 nm;

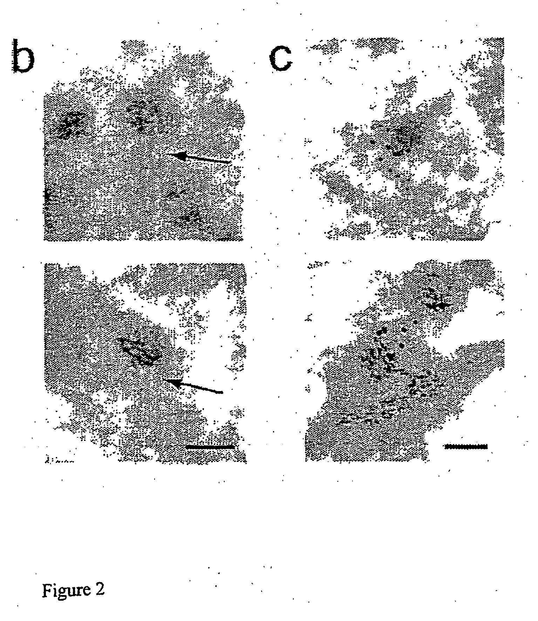

Figure 2 demonstrates results obtained in the comparative example. This figure is number 1 in the manuscript (Miralles et al, 2000).
In the figures are depicted fibers associated with BR particles in the nucleoplasm of the salivary gland cells. (b) Two examples of nucleoplasmic BR particles in a thin cryosection of a salivary gland stained with uranyl acetate and observed in the EM. The BR particles appear as dense granules with a diameter of ca 50nm. The arrows point at fibers that seem to contact the BR particle and extend into the surrounding nucleoplasm. (c) Two examples of fibers immunolabeled with a monoclonal antibody against a protein located in the fibers and gold-conjugated secondary antibody. The top panel shows a BR particle located in the nucleoplasm, far from both the BR gene and the nuclear envelope. Bars, 50 nm;

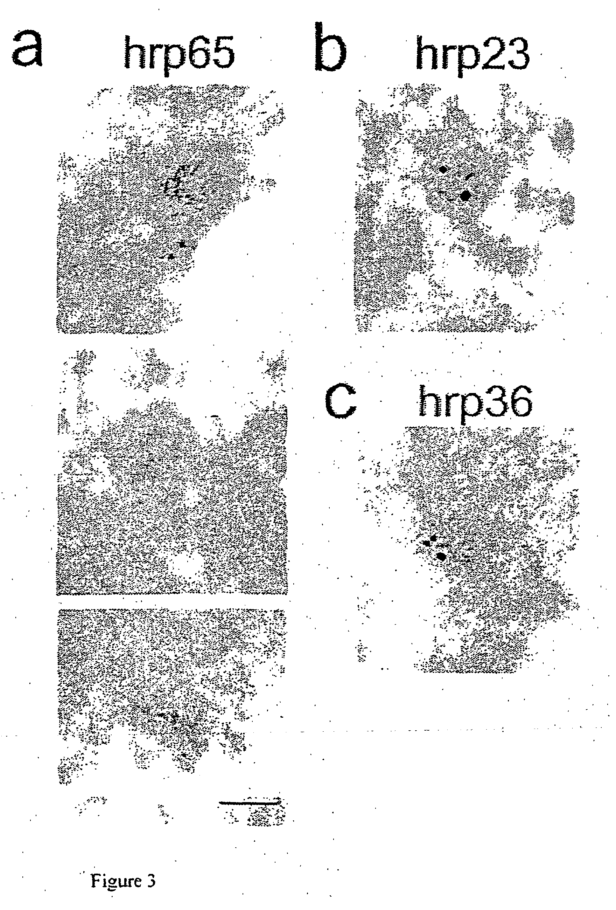

Figure 3 demonstrates results obtained in the comparative example. This figure is number 6 in the manuscript (Miralles et al, 2000). Localization of hrp65 in fibers. (a) Immuno-EM localization of hrp65 on thin cryosections of salivary glands. The sections were incubated with antibodies against hrp65, and with a gold-conjugated secondary antibody. Three examples of fibers (b and c) Immuno-EM localization of hrp23 and hrp36, respectively on thin cryosections of salivary glands. Bar, 50 nm. It should be noted that these figures (2 and 3) are two-dimensional representations and that the antigen designated by the gold marker is present somewhere around the gold within a circle with a radius of approximately 15 nm, but it cannot be localized with higher precision.

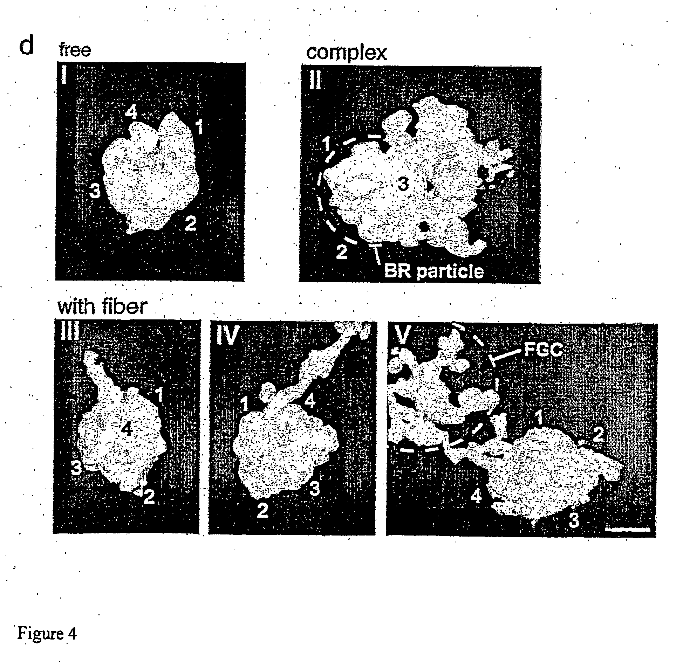

Figure 4d demonstrates results obtained in the comparative example. This figure is number 2d in the manuscript (Miralles et al, 2000). In this figure, a three dimensional reconstruction has been made. The figures I-V show BR particles to a higher resolution. It should be noted however, that specific components of the particles cannot be pinpointed due to lack of identification criteria. Bar, 20 nm.

### Definitions and abbreviations

- AR-: Affinity reagent
- PVA -: Polyvinyl alcohol
- EM -: Electron microscopy
- TEM -: Transmission electron microscopy
- EMT -: Electron microscope tomography
- COMET -: Constrained maximum entropy tomography
- 3D -: Three dimensional

As disclosed herein, the term "specimen" relates to a biological or chemical sample, such as a cell, tissue, or sample located in a container or test tube of any kind.

As disclosed herein, the term "affinity reagent" relates to molecules that bind specifically to certain epitopes. Such affinity reagents typically include antibodies, peptides, polypeptides, nucleotides, polynucleotides, drugs, candidate drugs, carbohydrates, fatty acids, proteins, or hormones.

As disclosed herein, the term "probe" can be used with the same meaning as "affinity reagent".

As disclosed herein, the term "direct labeling" relates to a method wherein the affinity reagent is labeled with a marker.

As disclosed herein, the term "electron dense" relates to a property of objects having a high density of electrons. Examples of such objects typically include iodine, ferritin, and heavy atoms such as gold, uranium or tungstate.

As disclosed herein, the term "epitope" relates to a specific binding site on a molecule recognized by an affinity reagent.

As disclosed herein, the term "indirect labeling" refers to a method where the marker is attached to a secondary affinity reagent that binds to the primary affinity reagent bound to the epitope of interest.

As disclosed herein, the term "low dose" relates to an electron dose not exceeding 35 e⁻/Å²/dataset

As disclosed herein, the term "3D reconstruction" relates to a method wherein a three dimensional image is reconstructed from several two dimensional images.

As disclosed herein, the term "data collection" relates to a method wherein the sample is exposed to electrons and the image produced recorded.

As disclosed herein, the term "tilt series" relates to a set of data collected from an electron tomography experiment. A tilt-series is a number of consecutive images taken in sequence. For each image, the camera is positioned at different angles with respect to the object.

As disclosed herein, the term "processing data" includes aligning, reconstructing and refining the collected data, as described in Skoglund et al., 1996b.

As disclosed herein, the term "chemical fixation" refers to a method wherein the sample is treated with compounds, typically glutaraldehyde or formaldehyde, that cross-link proteins and/or nucleic acids

As disclosed herein, the term "cryo" relates to low-temperature conditions where massive formation of ice crystals is prevented. This is well known to a person skilled in the art. An example of such a condition would be at a temperature below - 170°C at a pressure of 1 atmosphere, or - 100°C in the presence of 2.3M sucrose.

As disclosed herein, the terms "ultra-cryo-microtomy" or "cryo-microtomy" relates to a method wherein a sample is cut in thin (20-90nm) slices at cryo-conditions.

### Material and methods

### Isolation of salivary glands and chemical fixing

Salivary glands from fourth instar *C. tentans* larvae were fixed in 2% glutaraldehyde in 0.1 M sodium cacodylate buffer containing 0.05 M sucrose (pH 7.2) for 2 hours at 4°C and then washed 4×15 minutes in the same buffer, without glutaraldehyde.

### Isolation of chromosomes and antibody labeling

Salivary glands were fixed for 2 minutes in 2% paraformaldehyde in TKM (10 mM triethanolamine-HCl, pH 7.0, 100 mM KCl, and 1 mM MgCl₂) at 4°C, before isolation of chromosome IV by pipetting up and down with small glass pipettes (Björkroth et al., 1988; Kiseleva et al., 1994). Chromosome IV was then transferred to a siliconized object glass and fixed for 30 minutes in TKM containing 4% paraformaldehyde at room temperature before the immunoreaction was carried out as described by Sun et al., (1998). The chromosomes were postfixed in 2% glutaraldehyde in TKM for one hour. The non-immunolabeled chromosomes were directly fixed in glutaraldehyde in TKM after isolation. The RNA polymerase II CTD and all hnRNP and splicing factors that we have been able to test (two SR proteins, two hnRNP proteins, U1 snRNP and U2 snRNP) remain bound to the active BR3 gene segments during these conditions.

Goat anti-*Drosophila* RNA polymerase IIo antibody (i.e. the hyperphosphorylated CTD of the large subunit of RNA polymerase II), "gAPα-PCTD", was a generous gift from Dr Arno L. Greenleaf (Duke University Medical Center, USA). Mouse anti-U2 snRNP B" antibody was purchased from ICN Pharmaceuticals, Inc. Both antibodies are specific in Western blots. Secondary IgG antibodies conjugated with 6 nm gold particles were purchased from Jackson ImmunoResearch Labs, Inc.

### Embedding, staining and sectioning

Following glutaraldehyde fixation, all specimens (intact glands and isolated chromosomes) were dehydrated and embedded in the same way (Björkroth et al., 1988). The isolated chromosomes were embedded on top of a siliconized glass slide. The plastic with the isolated chromosomes was removed from the glass with a razor after cooling in liquid nitrogen. The area of interest was cut out, mounted and sectioned either into 60 nm or 100 nm sections. The sections were put onto EM grids (serial sections were ordered) and stained with 2% uranyl acetate in ethanol for 5 minutes. Colloidal 10 nm gold markers (Auroprobe EM GAD 1gG G19, Amersham), used as markers in the alignment, were added on top of the sections. The gold markers were diluted 1:3 and incubated with the grids for 8-10 minutes. The grids were finally washed 3 times in water and air-dried.

### Data collection

The tilt series (data set) of the chromosomes in sectioned nuclei were recorded manually on a Zeiss EM902 transmission electron microscope at 80 kV acceleration voltage using the inelastic scattering filter, after pre-irradiation of the preparations, resulting in an initial 20% shrinkage in the beam direction and prevention of any further shrinkage during the tilt series. A magnification of 32,000 times was used and every 5-degree from +60° to -60° was recorded, giving a total of 25 projections. Two extra zero-degree tilts were recorded, before and after the tilt series, to control for specimen radiation damage. Only approved tilt series were processed further, and the negatives were scanned with an Optronics P-1000 drum scanner with a 25 µm raster size.
The isolated chromosome tilt series, including immunolabeled chromosomes, were recorded on a Philips CEM200 FEG transmission electron microscope automatically, using the TVIPS (TVIPS GmbH, Gauting, Germany) detection system and the Emmenu software. This software controls the tilting, focusing, tracking and data collection. The acceleration voltage was 200 kV, the magnification 26,700 times and the pictures recorded directly on a 2048x2048 pixel CCD chip with a raster size of 14 µm. 61 pictures were recorded, every 2-degrees between +60° to -60°. The total dose for a full tilt series was less than 30 electrons/Å². No shrinkage can be detected during these conditions (Miralles et al., 2000).
The tilt series of chromosomes in situ were digitized at a pixel size of 7.8 Å and the tilt series of isolated chromosomes were digitized at a pixel size of 5.2 Å. The total size of the digitized area for the tilt series of chromosomes in sectioned nuclei was 1.25µm x 1.25 µm and for the isolated chromosomes 1.07µm x 1.07 µm.

### 3D Reconstruction

The data was processed (aligned, reconstructed and refined) using the ET software package version 3.4 (Skoglund et al., 1996b). All image processing was done on a Compaq workstation (500a) and a GS60 server.
10 nm colloidal gold markers were used for the alignment of the different projections. The average alignment error was less then 5 Å. The entire digitized *in situ* area was reconstructed using the filtered back-projection principle. The individual reconstructions were further refined using COMET (Skoglund et al., 1996a) to give a better fit to the experimental data, improve signal/noise ratio and to keep only significant densities. Finally, the reconstructed densities were lowpass-filtered to a homogenous resolution.

Areas of approximately 300 x 300 x 100 nm were first analyzed at 7.5-nm resolution. Selected structures were further reconstructed and analyzed at 5 nm. Bob (Ken Chin-Purcell, Minnesota Supercomputing Center Inc., freeware) was used for volume rendering and XTV (Skoglund et al., 1996b) and AVS/Express version 4.0 (Advanced Visual Systems, Inc) were both used for surface rendering.
The volumes of individual NTS complexes were measured by adding the voxels within a tailor-made envelope (only the density of interest was cut out using the XTV software). Only the voxels above a threshold density value, usually 2 standard deviations above average density, were added. The linear extension of gene segments from embedded isolated chromosomes compared to in situ sections, showed an approximately 20% shrinkage in the isolated chromosomes, most probably due to the different treatments of the specimens. The linear shrinkage corresponds to an isotropic shrinkage in volume of about 50% compared to the in situ sections.

### COMPARATIVE EXAMPLE OF PRIOR ART

In Miralles et al.; (2000), J. Cell Biol. 148, 271-282, are shown examples of information obtained prior to this invention. Figure 1 and 6 show typical examples of low-resolution images achieved without the use of COMET. The antibodies used in this example are not used in the context of a 3D reconstruction of the images and are of low resolution. In figure 2d is shown results achieved when COMET is used for 3D reconstruction of the images. In figure 2d, no antibody was used for identifying epitopes; therefore, it is impossible to identify specific components of the object.

### Immunological localization of the RNA polymerase II CTD and U2 snRNP B" protein

The locations of the CTD of RNA polymerase II and the U2 snRNP specific B" protein in the BR3 gene segments has been determined by immunological labeling.

Isolated chromosome IV was used in these experiments, because compared to sections of gland cells, the antibody reaction was more efficient.
Specific gold labeling of the active BR3 gene segments was obtained both for the anti-RNA polymerase II CTD antibody and the anti-U2 snRNP B" antibody. More than 99% (anti-U2 snRNP B" antibody) and 97% (anti-RNA polymerase II CTD antibody) of the gold particles were associated with the active gene segments and only very few particles were found in other regions (see Fig. 1 A). In the 3D reconstruction of the gold particles completely located within the section, more than 83 % (for the RNA polymerase II CTD antibody) and 97 % (for the anti-U2 snRNP B" antibody) had a specific connection to NTS complexes (see below). Using an unspecific antibody (data not shown), very few gold particles (less than 1% and 3% of the number seen with the anti-U2 snRNP B" antibody and anti-RNA polymerase II CTD antibody, respectively) were seen, and these gold particles were randomly distributed in relation to other parts of the chromosome.

The immuno gold labeling was clearly specific for the transcribing BR3 gene segments (Fig 1 A), but in two dimensions it was not possible to decide precisely which parts of the active gene segments were labeled (Fig. 1 B and D). The 3D reconstruction of the immunostained active BR3 gene enabled a high degree of precision in locating the antigens in the NTS complexes. In general, the combination of immunostaining and 3D reconstruction allows epitopes to be mapped, giving significantly improved possibilities of locating individual components in supra-molecular structures and in this way dissecting the composition of such structures.

An unambiguous thin electron-dense connection from most gold particles was found in the 3D reconstruction. This connection extended 10-20 nm from a gold particle and, with the U2 snRNP B" antibody, only the NTS complex was observed being labeled. Most of the connections were in this case to the part of the complex facing away from the chromatin axis (Fig. 1C). In addition, with the anti-RNA polymerase II CTD antibody, only labeling of the NTS complexes in the gene segments was observed. The chromatin axis between the NTS complexes was not labeled. This supports the interpretation from the structure of the active gene segments, that each NTS complex contains a nascent transcript and it follows that an RNA polymerase II is present in each NTS complex. Most connections between the gold particles attached to the anti-RNA polymerase II CTD antibodies and the NTS complex were observed at the region of the NTS complex that is closest to the chromatin axis (Fig. IE).

These immunolabeling experiments show that the CTD of the large subunit of RNA polymerase II and the U2 snRNP B" can both be localized to the NTS complexes along the chromatin axis. Furthermore, and of relevance to this invention, the data is of sufficiently high resolution to pinpoint the two components different locations within the complexes; the RNA polymerase II CTD closest to the chromatin axis and the U2 snRNP further inside the complex.

### References

Amero, S.A., Raychaudhuri, G, Cass, C.L., van Venrooij, W.J., Habets, W.J., Krainer, A.R., and Beyer, A.L. (1992) Independent deposition of heterogeneous nuclear ribonucleoproteins and small nuclear ribonucleoprotein particles at sites of transcription. Proc. Natl. Acad. Sci. USA 89, 8409-8413 .
Auer, J. Mol. Med., 78: 191-202, (2000)
Baurén, G., Jiang, W.-Q., Bernholm, K., Gu, F., and Wieslander, L. (1996) Demonstration of a dynamic, transcription dependent organization of pre-mRNA splicing factors in polytene nuclei. J. Cell Biol. 133, 929-941.
Bentley, D. (1999) Coupling RNA polymerase II transcription with pre-mRNA processing. Curr. Opin. Cell Biol. 11, 347-351.
Björkroth, B., Ericsson, C., Lamb, M.M., and Daneholt, B. (1988) Structure of the chromatin axis during transcription. Chromosoma 96, 333-340.
Clark, M.W., Goelz, S., and Abelson, J. (1988) Electron microscopic identification of the yeast spliceosome. EMBO J. 7, 3829-3836.
Cmarko, D., Verschure, P.J., Martin, T.E., Dahmus, M.E., Krause, S., Fu, X.-D., van Driel, R., and Fakan, S. (1999) Ultrastructural analysis of transcription and splicing in the cell nucleus after bromo-UTP microinjection. Mol. Biol. Cell. 10, 211-223.
Corden, J.L. and Patturajan, M. (1997) A CTD function linking transcription to splicing. Trends Biochem. Sci. 22, 413-416.
Henderson, R., The potential and limitations of neutrons, electrons and X-rays for atomic resolution microscopy of unstained biological molecules. Q Rev Biophys., (1995) May; 28(2): 171-93
Kiseleva, E., Wurtz, T., Visa, N., and Daneholt, B. (1994) Assembly and disassembly of spliceosomes along a specific pre-messenger RNP fiber. EMBO J. 13, 6052-6061.
Miralles, F., Öfverstedt, L.-Ö., Sabri, N., Aissouni, Y., Hellman, U., Skoglund, U. and Visa, N. (2000) Electron tomography reveals posttranscriptional binding of pre-mRNPs to specific fibers in the nucleoplasm. J. Cell Biol. 148, 271-282.
Neugebauer, K.M., and Roth, M.B. (1997a) Distribution of pre-mRNA splicing factors at sites of RNA polymerase II transcription. Genes & Dev. 11, 1148-1159.
Neugebauer, K.M., and Roth, M.B. (1997b) Transcription units as RNA processing units. Genes & Dev. 11: 3279-3285.
Paulsson, G., Lendahl, U., Galli, J., Ericsson, C., and Wieslander, L. (1990) The Balbiani Ring 3 gene in Chironomus tentans has a diverged repetitive structure split by many introns. J. Mol. Biol. 211, 331-349.
Puvion, E., and Puvion-Dutilleul, F. (1996) Ultrastructure of the nucleus in relation to transcription and splicing: role of perichromatin fibrils and interchromatin granules. Exp. Cell Res. 229, 217-225.
Raab et al. Antibody recognition imaging by force microscopy. Nature Biotechnology, (1999), Sept., 17: 902-905
Reed, R., Griffith, J., and Maniatis, T. (1988) Purification and visualization of native spliceosomes. Cell 53, 949-961.
Shuman, S. (1997) Origins of mRNA identity: Capping enzymes bind to the phosphorylated C-terminal domain of RNA polymerase II. Proc. Natl. Acad. Sci. U.S.A. 94, 12758-12760.
Skoglund, U., Öfverstedt, L.-G., Burnett, R, and Bricogne, G. (1996a) Maximum-entropy three- dimensional reconstruction with deconvolution of the contrast transfer function, a test application with adenovirus. J. Struct. Biol. 117, 173-188.
Skoglund, U., Öfverstedt, L.-G., and Daneholt, B. (1996b) Procedures for three-dimensional reconstruction from thin slices with electron tomography, In RNP particles, Splicing and Immunodiseases. Springer Lab Manual, J.Schenkel, ed. (Springer-Verlag, Heidelberg), pp. 72-94.
Steinmetz, E.J. (1997) Pre-mRNA processing and the CTD of RNA polymerase II: The tail that wags the dog. Cell 89, 491-494.
Sun, X., Alzha.nova-Ericsson, A., Visa, N., Aissouni, Y., Zhao, J. and Daneholt, B. (1998) The hrp23 protein in the Balbiani ring pre-mRNP particles is released just before or at the binding of the particles to the nuclear pore complex. J. Cell Biol. 142, 1-13.
Valle et al. Selection of antibody probes to correlate protein sequence domains with their structural distribution. Protein Science, (1999) 8: 883-889
Wetterberg, I., Baurén, G., and Wieslander, L. (1996) The intranuclear site of excision of each intron in the Balbiani Ring 3 pre-mRNA is influenced by the time remaining to transcription termination and different excision efficiencies for the various introns. RNA 2, 641-651.
Wu, Z., Murphy, C., Callan, H.G. and Gall, J.G. (1993) Small nuclear ribonuceloproteins and heterogeneous nuclear ribonucleoproteins in the amphibian germinal vesicle: Loops, spheres, and snurposomes. J. Cell Biol. 113, 465-483.
Zhang, G., Taneja, K.L., Singer, R.H., and Green, M.R., (1994) Localization of pre-mRNA splicing in mammalian nuclei. Nature 372, 809-812.

## Claims

1. Method for localizing and visualizing specific epitopes located on a molecule in a specimen, comprising the steps of;
(a) performing affinity labeling by binding a first affinity reagent to specific epitopes of said molecule in said specimen;
(b) fixing the spatial relationships between said molecule and other components of the specimen by freezing and/or embedding;
(c) applying the sample on an electron microscopy grid;
(d) exposing the specimen to a dose not exceeding 35 e⁻/Å² of electron radiation in a transmission electron microscope;
(e) collecting data from a tilt-series;
(f) analyzing the collected data to reconstruct, in three dimensions, the structures of individual molecules;
(g) refining said reconstructions using Constrained Maximum Entropy Tomography (COMET) to localize and visualize specific epitopes of said specimen.

2. The method according to claim 1, whereby said first affinity reagent directed against two or more epitopes on said molecule is employed for affinity labeling.

3. The method according to any of the preceding claims, whereby said first affinity reagent is labeled with an electron-dense marker.

4. The method according to any of the preceding claims, whereby a second affinity reagent is bound to said affinity reagent, which is bound to said molecule.

5. The method according to any of the preceding claims, whereby said first affinity reagent is injected into a cell or tissue in which said molecule is located.

6. The method according to claim 5, whereby said cell or tissue containing said first affinity reagent is sliced into thin sections of a thickness in the range of 25 nm-1 µm.

7. The method according to claim 1, whereby said specimen is sliced in sections of a thickness in the range of 25 nm-1 µm prior to affinity labeling.

8. The method according to any of the preceding claims, in which the specimen is frozen after affinity labeling and prior to exposing said specimen to said dose of electron radiation.

9. The method according to claim 8, in which the specimen is sliced and embedded.

10. The method according to any of the preceding claims, in which the spatial relationships between said molecules and other components of the specimen are fixed by chemical fixation prior to freezing and/or embedding.

11. The method according to any of the preceding claims, further comprising fixing the spatial relationships between components of the sample by embedding, and/or freezing prior to slicing.

12. The method according to any of the preceding claims, in which the specimen is frozen after being placed on said electron microscopy grid and prior to exposing to said -dose of electron radiation

13. The method according to any of the above claims in which the specimen is stained with heavy atoms prior to data collection.

## Patentansprüche

1. Verfahren zum Lokalisieren und Visualisieren spezifischer Epitope, die sich auf einem Molekül in einer Probe befinden, die folgenden Schritte umfassend:
(a) Durchführen einer Affinitätskennzeichnung, indem ein erstes Affinitätsreagens an spezifische Epitope des Moleküls in der Probe gebunden wird;
(b) Festlegen der räumlichen Verhältnisse zwischen dem Molekül und anderen Bestandteilen der Probe durch Einfrieren und/oder Einbetten;
(c) Aufbringen der Probe auf ein Elektronenmikroskopiegitter;
(d) Bestrahlen der Probe mit einer Dosis, die 35 e⁻/Å² Elektronenstrahlung nicht überschreitet, in einem Transmissionselektronenmikroskop;
(e) Sammeln von Daten aus einer Kippreihe;
(f) Analysieren der gesammelten Daten, um die Strukturen einzelner Moleküle in drei Dimensionen zu rekonstruieren;
(g) Verfeinern der Rekonstruktionen unter Verwendung von Constrained Maximum Entropy Tomography (COMET), um die spezifischen Epitope der Probe zu lokalisieren und visualisieren.

2. Verfahren nach Anspruch 1, wobei das erste Affinitätsreagens, das sich gegen zwei oder mehr Epitope auf dem Molekül richtet, zur Affinitätskennzeichnung verwendet wird.

3. Verfahren nach einem der vorhergehenden Ansprüche, wobei das erste Affinitätsreagens mit einem elektronendichten Marker **gekennzeichnet** wird.

4. Verfahren nach einem der vorhergehenden Ansprüche, wobei ein zweites Affinitätsreagens an das Affinitätsreagens gebunden wird, das an das Molekül gebunden ist.

5. Verfahren nach einem der vorhergehenden Ansprüche, wobei das erste Affinitätsreagens in eine Zelle oder in Gewebe injiziert wird, in der bzw. dem sich das Molekül befindet.

6. Verfahren nach Anspruch 5, wobei die Zelle oder das Gewebe, die bzw. das das erste Affinitätsreagens enthält, in dünne Abschnitte mit einer Dicke im Bereich von 25 nm - 1 µm geschnitten wird.

7. Verfahren nach Anspruch 1, wobei die Probe vor der Affinitätskennzeichnung in Abschnitte mit einer Dicke im Bereich von 25 nm - 1 µm geschnitten wird.

8. Verfahren nach einem der vorhergehenden Ansprüche, wobei die Probe nach der Affinitätskennzeichnung und vor dem Bestrahlen mit der Elektronenstrahlungsdosis eingefroren wird.

9. Verfahren nach Anspruch 8, wobei die Probe geschnitten und eingebettet wird.

10. Verfahren nach einem der vorhergehenden Ansprüche, wobei die räumlichen Verhältnisse zwischen den Molekülen und anderen Bestandteilen der Probe vor dem Einfrieren und/oder Einbetten durch chemisches Fixieren fixiert werden.

11. Verfahren nach einem der vorhergehenden Ansprüche, darüber hinaus vor dem Zerschneiden ein Festlegen der räumlichen Verhältnisse zwischen Bestandteilen der Probe durch Einbetten und/oder Einfrieren umfassend.

12. Verfahren nach einem der vorhergehenden Ansprüche, wobei die Probe, nachdem sie auf das Elektronenmikroskopiegitter gelegt wurde, und bevor sie mit der Elektronenstrahlung bestrahlt wird, eingefroren wird.

13. Verfahren nach einem der vorhergehenden Ansprüche, wobei die Probe vor der Datenerfassung mit schweren Atomen angefärbt wird.

## Revendications

1. Méthode permettant de localiser et visualiser des épitopes spécifiques portés sur une molécule d'un prélèvement, et comportant les séquences suivantes :
(a) effectuer un étiquetage d'affinité en liant un premier réactif d'affinité aux épitopes spécifiques de ladite molécule dans ledit prélèvement ;
(b) fixer les relations spatiales entre ladite molécule et d'autres composants du prélèvement, par congélation et/ou fixation
(c) appliquer le prélèvement sur la grille d'un microscope électronique ;
(d) exposer le prélèvement à une dose de rayonnement ne dépassant pas 35 e/A² à l'aide d'un microscope électronique en transmission ;
(e) recueillir les données d'une tilt-series
(f) analyser les données recueillies pour reconstruire, en 3D, les structures individuelles de chaque molécule ;
(g) raffiner lesdites reconstructions à l'aide de COMET (tomographie à maximum d'entropie sous contrainte), afin de localiser et visualiser les épitopes spécifiques dudit prélèvement.

2. Méthode selon la revendication 1, dans laquelle ledit premier réactif d'affinité dirigé contre deux épitopes, ou plus, sur ladite molécule est utilisé pour un étiquetage d'affinité

3. Méthode selon n'importe laquelle des revendications précédentes dans laquelle ledit premier réactif d'affinité est étiqueté avec un marqueur dense aux électrons ;

4. Méthode selon n'importe laquelle des revendications précédentes, dans laquelle un second réactif d'affinité est lié audit réactif d'affinité, lui-même lié à ladite molécule ;

5. Méthode selon n'importe laquelle des revendications précédentes, dans laquelle ledit premier réactif d'affinité est injecté dans une cellule ou un tissu dans lequel est localisée ladite molécule.

6. Méthode selon la revendication 5 dans laquelle ladite cellule ou ledit tissu comportant ledit premier réactif d'affinité est découpé en fines lamelles d'une épaisseur variant entre 25 nm-1µm ;

7. Méthode selon la revendication 1, dans laquelle le prélèvement est découpé en fines lamelles d'une épaisseur variant entre 25 nm-1µm avant l'étiquetage d'affinité ;

8. Méthode selon n'importe laquelle des revendications précédentes, dans laquelle le prélèvement est congelé après l'étiquetage d'affinité et avant l'exposition dudit prélèvement à ladite dose de rayonnements électroniques.

9. Méthode selon la revendication 8, dans laquelle le prélèvement est découpé en lamelles et fixé.

10. Méthode selon n'importe laquelle des revendications précédentes, dans laquelle les relations spatiales entre lesdites molécules et les autres composants du prélèvement sont figées par fixation chimique avant congélation et/ou fixation.

11. Méthode selon n'importe laquelle des revendications précédentes, comprenant en plus la fixation de la relation spatiale entre les composants du prélèvement par fixation et/ou congélation avant la découpe en lamelles.

12. Méthode selon n'importe laquelle des revendications précédentes dans laquelle le prélèvement est congelé après avoir été placé sur la grille dudit microscope électronique et avant son exposition à ladite dose de rayonnements électroniques ;

13. Méthode selon n'importe laquelle des revendications ci-dessus, dans laquelle le prélèvement est coloré avec des atomes lourds avant le recueil des données.
